(19) **Europäisches Patentamt / European Patent Office / Office européen des brevets**

(11) **EP 1 429 738 B1**

(12) **EUROPEAN PATENT SPECIFICATION**

(45) Date of publication and mention
of the grant of the patent:
**31.10.2007 Bulletin 2007/44**

(21) Application number: **02783988.5**

(22) Date of filing: **28.09.2002**

(51) Int Cl.:
***A61K 9/20*** *(2006.01)*

(86) International application number:
**PCT/US2002/031062**

(87) International publication number:
**WO 2003/026626 (03.04.2003 Gazette 2003/14)**

(54) **MODIFIED RELEASE DOSAGE FORMS**

DARREICHUNGSFORMEN ZUR MODIFIZIERTEN FREISETZUNG

FORME PHARMACEUTIQUE A LIBERATION MODIFIEE

(84) Designated Contracting States:
**AT BE BG CH CY CZ DE DK EE ES FI FR GB GR IE IT LI LU MC NL PT SE SK TR**

(30) Priority: **28.09.2001 US 966939**
**28.09.2001 US 966509**
**28.09.2001 US 966497**
**28.09.2001 US 967414**
**28.09.2001 US 966450**

(43) Date of publication of application:
**23.06.2004 Bulletin 2004/26**

(73) Proprietor: **McNEIL-PPC, Inc.**
**Skillman, NJ 08558 (US)**

(72) Inventors:
• **LEE, Der-Yang**
**Flemington, NJ 08822 (US)**

• **LI, Shun-Por**
**Lansdale, PA 19446 (US)**
• **PARIKH, Narendra**
**Long Valley, NJ 07853 (US)**
• **SOWDEN, Harry, S.**
**Glenside, PA 19038 (US)**
• **THOMAS, Martin**
**Lake Worth, FL 33467 (US)**
• **WYNN, David**
**Abington, PA 19001 (US)**

(74) Representative: **Fisher, Adrian John et al**
**CARPMAELS & RANSFORD**
**43-45 Bloomsbury Square**
**London WC1A 2RA (GB)**

(56) References cited:
**EP-A- 0 279 682**      **US-A- 5 100 675**
**US-A- 5 415 868**      **US-A- 5 558 879**
**US-A- 5 807 579**      **US-A- 5 824 338**

EP 1 429 738 B1

**Description**

1. <u>Field of the Invention</u>

This invention relates to modified release dosage forms such as modified release pharmaceutical compositions. More particularly, this invention relates to modified release dosage forms having a core containing at least one active ingredient and a shell surrounding the core, in which the shell provides a delay of greater than one hour for the onset of dissolution of the active ingredient upon contacting of the dosage form with a liquid medium such as water or gastrointestinal fluids, and the delay is independent of the pH of the liquid medium.

2. <u>Background Information</u>

Modified release pharmaceutical dosage forms have long been used to optimize drug delivery and enhance patient compliance, especially by reducing the number of doses of medicine the patient must take in a day. For this purpose, it is often desirable to modify the rate of release of a drug (one particularly preferred type of active ingredient) from a dosage form into the gastro-intestinal (g.i.) fluids of a patient, especially to slow the release in order to provide prolonged action of the drug in the body.

The rate at which an orally delivered pharmaceutical active ingredient reaches its site of action in the body depends on a number of factors, including the rate and extent of drug absorption through the gastro-intestinal (g.i.) mucosa. To be absorbed into the circulatory system (blood), the drug must first be dissolved in the g.i. fluids. For many drugs, diffusion across the g.i. membranes is relatively rapid compared to dissolution. In these cases, the dissolution of the active ingredient is the rate limiting step in drug absorption, and controlling the rate of dissolution allows the formulator to control the rate of drug absorption into the circulatory system of a patient.

An important objective of modified release dosage forms is to provide a desired blood concentration versus time (pharmacokinetic, or PK) profile for the drug. Fundamentally, the PK profile for a drug is governed by the rate of absorption of the drug into the blood, and the rate of elimination of the drug from the blood. The type of PK profile desired depends, among other factors, on the particular active ingredient, and physiological condition being treated.

One desirable PK profile for a number of drugs and conditions, is achieved by a dosage form that delivers a delayed release dissolution profile, in which the release of drug from the dosage form is delayed for a pre-determined time after ingestion by the patient. The delay period ("lag time") can be followed either by prompt release of the active ingredient ("delayed burst"), or by sustained (prolonged, extended, or retarded) release of the active ingredient ("delayed then sustained").

A particularly desirable type of delayed release PK profile is a "pulsatile" profile, in which for example, a first dose is delivered immediately, followed by a delay corresponding approximately to the time during which a therapeutic concentration of the first dose is maintained in the blood, followed by either prompt or sustained release of a subsequent dose of the same drug.

A particularly challenging aspect of the design of delayed release systems involves the predictability and repeatability of the lag time *in vivo.* Physiological systems, e.g. the human g.i. tract, have a high degree of inter- and intra-subject variability, for example in intestinal motility and pH. For the purpose of repeatability and predictability, it is desirable to have a delayed release mechanism that is independent of the pH of the environment in which the dosage form must release drug.

Well known mechanisms by which a dosage form (or drug delivery system) can deliver drug at a modified rate (e.g. delayed, pulsatile, sustained, prolonged, extended or retarded release) include diffusion, erosion, and osmosis.

One classic diffusion-controlled release system comprises active ingredient, distributed throughout an insoluble porous matrix through which the active ingredient must diffuse in order to be absorbed into the bloodstream of the patient. The amount of drug release (M) at a given time at sink conditions (i.e. drug concentration at the matrix surface is much greater than drug concentration in the bulk solution) depends on the area (A) of the matrix, the diffusion coefficient (D), the porosity (E) and tortuosity (T) of the matrix, the drug solubility (Cs) in the dissolution medium, time (t) and the drug concentration (Cp) in the dosage form:

$$M = A\,(DE/T(2Cp - ECs)\,(Cs)\,t)^{1/2}$$

It will be noted in the above relationship that the amount of drug released is generally proportional to the square root of time. Assuming factors such as matrix porosity and tortuosity are constant within the dosage form, a plot of amount of drug released versus the square root of time should be linear.

A commonly used erosion-controlled release system comprises a "matrix" throughout which the drug is distributed. The matrix typically comprises a material which swells at the surface, and slowly dissolves away layer by layer, liberating drug as it dissolves. The rate of drug release, dM/dt, in these systems depends on the rate of erosion (dx/dt) of the matrix, the concentration profile in the matrix, and the surface area (A) of the system:

$$dM/dt = A \{dx/dt\} \{f(C)\}$$

Again, variation in one or more terms, such as surface area, typically lead to a non-constant release rate of drug. In general, the rate of drug release from erosion-controlled release systems typically follows first order kinetics.
Another type of erosion controlled delivery system utilizes materials which swell and dissolve slowly by surface erosion are additionally useful for providing a delayed release of pharmaceutical active ingredient. Delayed release is useful, for example in pulsatile or repeat action delivery systems, in which an immediate release dose is delivered, followed by a pre-determined lag time before a subsequent dose is delivered from the system. In these systems, the lag time ($T_1$) depends on the thickness (h) of the erodible layer, and the rate of erosion (dx/dt) of the matrix, which in turn depends on the swelling rate and solubility of the matrix components:

$$T_1 = h \ (dx/dt)$$

The cumulative amount of drug (M) released from these systems at a given time generally follows the equation:

$$M = (dM/dt) (t - T_1)$$

where dM/dt is generally described by either the diffusion-controlled or erosion-controlled equations above, and $T_1$ is the lag time.
It is often practical to design dosage forms that use a combination of the above mechanisms to achieve a particularly desirable release profile for a particular active ingredient.
Current delayed-release systems are limited by the available methods for manufacturing them, as well as the materials that are suitable for use with the current methods. A shell, or coating, which confers modified release properties, is typically applied via conventional methods, such as for example, spray-coating in a coating pan. Pan-coating produces a single shell which essentially surrounds the core. The single shell is inherently limited in its functionality. It is possible via pan-coating to apply multiple concentric shells, each with a different functionality, however such systems are limited in that the outer shell must first dissolve before the functionality conferred by each successive layer can be realized. Additionally, the coating compositions that can be applied via spraying are limited by their viscosity. Spray-coating methods suffer the further limitations of being time-intensive and costly. One well-known and commonly used design for providing delayed release of a drug employs an enteric coating material, either on particles containing the drug or on the surface of a dosage form. Enteric materials are generally selected from polymer systems which are soluble only in fluid environments with a certain pH range, higher than that of typical gastric fluid, for example pH greater than 5.5, greater than pH 6.0, or greater than pH 7.0. While these systems may be useful for protecting certain acid-labile active ingredients from gastric fluids, or for protecting the stomach lining from damage by certain active ingredients, they are limited in their applicability to programmed time-delay systems due to variability in gastrointestinal pH and motility.
The pH-independent delay of drug release has been achieved by conventional spray-coating methods. For example, G. Maffione et al., "High-Viscosity HPMC as a Film-Coating Agent," Drug Development and Industrial Pharmacy (1993) 19(16), pp. 2043-2053, describes a core or tablet matrix, surrounded by a shell or coating, which provides a delayed burst dissolution profile. Coating levels were 12.5-25% of the weight of the core. A preferred coating formula employs a swellable film-former dispersed in non-aqueous solvent. Low polymer concentrations (5-10%), and the use of ethanol as a "non-solvent" were required for sprayability.
It is also known, via pan coating, to deliver a first dose of active ingredient from a coating, and a second dose of active ingredient from a core. U.S. Patent No. 4,576,604, for example, discloses an osmotic device (dosage form) comprising a drug compartment surrounded by a wall (coating) in which the coating may comprise an immediate release dose of drug, and the inner drug compartment may comprise a sustained release dose of drug.
Alternately, conventional controlled release systems may be prepared by compression, to produce either multiple stacked layers, or core and shell configurations. Modified release dosage forms prepared via compression are exemplified in U.S. Patent Nos. 5,738,874 and 6,294,200, and WO 99/51209.

It is possible via compression-coating to produce a pH-independent time delayed drug release. U.S. Patent No. 5,464,633 discloses delayed-release dosage forms in which an external coating layer was applied by a compression coating process. The coating level ranged from 105 percent to 140 percent of the weight of the core in order to yield product with the desired time delayed profile.

Compression-coated dosage forms are limited by the shell thickness and shell composition. Gunsel et al., "Compression-coated and layer tablets" in Pharmaceutical Dosage Forms - Tablets edited by H. A. Lieberman, L. Lachman, and J. B. Schwartz, (2nd ed., rev. and expanded Marcel Dekker) Inc., p. 247-284, for example, discloses the thickness of compression coated shells is typically between 800 and 1200 microns.

Dosage forms comprising at least one active ingredient, a core and a molded shell are disclosed in US-A-5 415 868, US-A-5 824 338, US-A-5 100 675, US-A-5 558 879, US-A-5 807 579 and EP-A- 0 279 682.

It is one object of this invention to provide a dosage form having a core which contains at least one active ingredient and a shell surrounding the core, in which the shell has a weight of at least 50 percent of the weight of the core, the shell provides a delay of greater than one hour for the onset of dissolution of the active ingredient upon contacting of the dosage form with a liquid medium, and the delay is independent of the pH of the liquid medium. Other objects, features and advantages of this invention will be apparent to those skilled in the art from the following detailed description of the invention.

SUMMARY OF THE INVENTION

The dosage form of this invention comprises:

(a) a core comprising at least one active ingredient; and
(b) a molded shell which surrounds the core, wherein the shell provides a predetermined time delay of greater than one hour for the onset of dissolution of the active ingredient upon contacting of the dosage form with a liquid medium and the delay is independent of the pH of the liquid medium.

The weight of the shell is at least 50 percent of the weight of the core.

The shell also has a thickness from 500 to 4000 microns; it has a pore volume of less than 0.02cm$^3$/g in the pore diameter range of 0.5 to 5.0 microns (as defined herein); and it comprises at least 30% of a thermal reversible carrier, selected from polyethylene glycol, polyethylene oxide and copolymers and combinations thereof.

In one embodiment, the shell has a surface gloss of at least about 150 gloss units.

In another embodiment, the shell additionally comprises at least one active ingredient which may be the same or different than the active ingredient contained in the core.

In another embodiment, the dosage form additionally comprises an outer coating which covers at least a portion of the shell, and the outer coating comprises at least one active ingredient which may be the same or different than the active ingredient contained in the core.

In another embodiment, the core is a compressed tablet.

In another embodiment, the core comprises coated particles of at least one active ingredient.

In another embodiment, the core is made by molding.

In another embodiment, the core is substantially free of pores having a diameter of 0.5 to 5.0 microns.

In another embodiment, the core comprises at least about 30 weight percent of a thermal-reversible carrier.

In another embodiment, the core comprises a release-modifying excipient.

In another embodiment, the shell is not a compression coating applied to the core.

In another embodiment, the dosage form provides an immediate release of at least one active ingredient, followed by a delay of at least about 1 hour, followed by a burst release of at least one active ingredient.

In another embodiment, the shell is prepared using a solvent-free molding process.

In another embodiment, the shell comprises up to 55% by weight of a swellable, erodible hydrophilic material.

In another embodiment, the shell is prepared using a solvent-free molding process.

In another embodiment, the shell comprises up to 55% by weight of a release-modifying excipient.

In another embodiment, the dosage form provides a delayed burst release profile of the active ingredient.

In another embodiment, the dosage form provides a delayed and sustained release profile of the active ingredient.

In another embodiment, the dosage form provides a pulsatile release profile of the active ingredient.

In another embodiment, the core or portion thereof further comprises shellac at a level of about 5 to about 15 weight percent of the core or portion thereof.

In another embodiment, the shell or portion thereof further comprises shellac at a level of about 5 to about 15 weight percent of the shell or portion thereof.

In another embodiment, the release-modifying excipient is a swelling cross-linked polymer.

In another embodiment, the swelling cross-linked polymer is croscarmellose sodium.

In another embodiment, the shell further comprises a plasticizer.

In another embodiment, the plasticizer is tributyl citrate.

BRIEF DESCRIPTION OF THE DRAWINGS

Fig. 1 depicts a cross-sectional view of one embodiment of this invention.

Fig. 2 depicts the percent release of active ingredient vs. hours for the dosage form of Example 1.

Fig. 3 depicts the percent release of active ingredient vs. hours for the dosage form of Example 2.

Fig. 4 depicts the percent release of active ingredient vs. hours for the dosage form of Example 3.

DETAILED DESCRIPTION OF THE INVENTION

As used herein, the term "dosage form" applies to any solid object, semi-solid, or liquid composition designed to contain a specific pre-determined amount (i.e. dose) of a certain ingredient, for example an active ingredient as defined below. Suitable dosage forms may be pharmaceutical drug delivery systems, including those for oral administration, buccal administration, rectal administration, topical or mucosal delivery, or subcutaneous implants, or other implanted drug delivery systems; or compositions for delivering minerals, vitamins and other nutraceuticals, oral care agents, flavorants, and the like. Preferably the dosage forms of the present invention are considered to be solid, however they may contain liquid or semi-solid components. In a particularly preferred embodiment, the dosage form is an orally administered system for delivering a pharmaceutical active ingredient to the gastro-intestinal tract of a human.

The active ingredient employed in the dosage forms of this invention may be found within the core, the shell or a combination thereof. Suitable active ingredients for use in this invention include for example pharmaceuticals, minerals, vitamins and other nutraceuticals, oral care agents, flavorants and mixtures thereof. Suitable pharmaceuticals include analgesics, anti-inflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, diuretics, expectorants, gastrointestinal agents, migraine preparations, motion sickness products, mucolytics, muscle relaxants, osteoporosis preparations, oral contraceptives, polydimethylsiloxanes, respiratory agents, sleep-aids, urinary tract agents and mixtures thereof.

Suitable oral care agents include breath fresheners, tooth whiteners, antimicrobial agents, tooth mineralizers, tooth decay inhibitors, topical anesthetics, mucoprotectants, and the like.

Suitable flavorants include menthol, peppermint, mint flavors, fruit flavors, chocolate, vanilla, bubblegum flavors, coffee flavors, liqueur flavors and combinations and the like.

Examples of suitable gastrointestinal agents include antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, cascara sagrada, danthron, senna, phenolphthalein, aloe, castor oil, ricinoleic acid, and dehydrocholic acid, and mixtures thereof; H2 receptor antagonists, such as famotadine, ranitidine, cimetadine, nizatidine; proton pump inhibitors such as omeprazole or lansoprazole; gastrointestinal cytoprotectives, such as sucraflate and misoprostol; gastrointestinal prokinetics, such as prucalopride, antibiotics for H. pylori, such as clarithromycin, amoxicillin, tetracycline, and metronidazole; antidiarrheals, such as diphenoxylate and loperamide; glycopyrrolate; antiemetics, such as ondansetron, analgesics, such as mesalamine.

In one embodiment of the invention, the active ingredient may be selected from bisacodyl, famotadine, ranitidine, cimetidine, prucalopride, diphenoxylate, loperamide, lactase, mesalamine, bismuth, antacids, and pharmaceutically acceptable salts, esters, isomers, and mixtures thereof.

In another embodiment, the active ingredient is selected from analgesics, antiinflammatories, and antipyretics, e.g. non-steroidal anti-inflammatory drugs (NSAIDs), including propionic acid derivatives, e.g. ibuprofen, naproxen, ketoprofen and the like; acetic acid derivatives, e.g. indomethacin, diclofenac, sulindac, tolmetin, and the like; fenamic acid derivatives, e.g. mefanamic acid, meclofenamic acid, flufenamic acid, and the like; biphenylcarbodylic acid derivatives, e.g. diflunisal, flufenisal, and the like; and oxicams, e.g. piroxicam, sudoxicam, isoxicam, meloxicam, and the like. In a particularly preferred embodiment, the active ingredient is selected from propionic acid derivative NSAID, e.g. ibuprofen, naproxen, flurbiprofen, fenbufen, fenoprofen, indoprofen, ketoprofen, fluprofen, pirprofen, carprofen, oxaprozin, pranoprofen, suprofen, and pharmaceutically acceptable salts, derivatives, and combinations thereof. In a particular embodiment of the invention, the active ingredient may be selected from acetaminophen, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof.

In another embodiment of the invention, the active ingredient may be selected from pseudoephedrine, phenylpropanolamine, chlorpheniramine, dextromethorphan, diphenhydramine, astemizole, terfenadine, fexofenadine, loratadine, desloratadine, cetirizine, mixtures thereof and pharmaceutically acceptable salts, esters, isomers, and mixtures thereof.

Examples of suitable polydimethylsiloxanes, which include, but are not limited to dimethicone and simethicone, are those disclosed in United States Patent Nos. 4,906,478, 5,275,822, and 6,103,260, the contents of each is expressly incorporated herein by reference. As used herein, the term "simethicone" refers to the broader class of polydimethylsiloxanes, including but not limited to simethicone and dimethicone.

The active ingredient or ingredients are present in the dosage form in a therapeutically effective amount, which is an amount that produces the desired therapeutic response upon oral administration and can be readily determined by one skilled in the art. In determining such amounts, the particular active ingredient being administered, the bioavailability characteristics of the active ingredient, the dose regime, the age and weight of the patient, and other factors must be considered, as known in the art. Typically, the dosage form comprises at least about 5 weight percent, preferably, the dosage form comprises at least about 20 weight percent of the active ingredient. In one preferred embodiment, the core comprises at least about 25 weight percent (based on the weight of the core) of the active ingredient.

The active ingredient or ingredients may be present in the dosage form in any form. For example, the active ingredient may be dispersed at the molecular level, e.g. melted or dissolved, within the dosage form, or may be in the form of particles, which in turn may be coated or uncoated. If the active ingredient is in form of particles , the particles (whether coated or uncoated) typically have an average particle size of about 1-2000 microns. In one preferred embodiment, such particles are crystals having an average particle size of about 1-300 microns. In another preferred embodiment, the particles are granules or pellets having an average particle size of about 50-2000 microns, preferably about 50-1000 microns, most preferably about 100-800 microns.

In certain embodiments of the invention, at least a portion of the active ingredient may be coated with a release-modifying coating, as known in the art. This advantageously provides an additional tool for optimizing the release profile of the active ingredient from the dosage form Examples of suitable release modifying coatings are described in U.S. Patent Nos. 4,173,626; 4,863,742; 4,980,170; 4,984,240; 5,286,497, 5,912,013; 6,270,805; and 6,322,819. Commercially available modified release coated active particles may also be employed. Accordingly, all or a portion of one or more active ingredients may be coated with a release-modifying material.

In certain other embodiments of the invention, a further degree of flexibility in designing the dosage forms of the present invention can be achieved through the use of an additional outer coating overlaying the shell. The additional outer coating may be applied by known methods, for example by spraying, dipping, printing, roller coating, compression, or by molding. In such embodiments, the dosage form of the invention comprises a core containing at least one active ingredient; a molded shell which surrounds the core, wherein the shell provides a predetermined time delay of greater than one hour for the onset of dissolution of the active ingredient upon contacting of the dosage form with a liquid medium and the delay is independent of the pH of the liquid medium; and an outer coating which covers at least a portion of the shell. In one particularly preferred embodiment, the dosage form is a pulsatile drug delivery system, in which the outer coating comprises an active ingredient, which is released immediately (i.e. the dissolution of the active ingredient from the outer coating conforms to USP specifications for immediate release dosage forms of the particular active ingredient employed).

In embodiments in which it is desired for the active ingredient to be absorbed into the systemic circulation of an animal, the active ingredient or ingredients are preferably capable of dissolution upon contact with a fluid such as water, stomach acid, intestinal fluid or the like. In one embodiment, the dissolution characteristics of at least one active ingredient meets USP specifications for immediate release tablets containing the active ingredient. For example, for acetaminophen tablets, USP 24 specifies that in pH 5.8 phosphate buffer, using USP apparatus 2 (paddles) at 50 rpm, at least 80% of the acetaminophen contained in the dosage form is released therefrom within 30 minutes after dosing, and for ibuprofen tablets, USP 24 specifies that in pH 7.2 phosphate buffer, using USP apparatus 2 (paddles) at 50 rpm, at least 80% of the ibuprofen contained in the dosage form is released therefrom within 60 minutes after dosing. See USP 24, 2000 Version, 19 - 20 and 856 (1999). In embodiments in which at least one active ingredient is released immediately, the immediately released active ingredient is preferrably contained in the shell or on the surface of the shell, e.g. in a further coating residing upon at least a portion of the shell. In another embodiment, the dissolution characteristics of at least one active ingredient are modified: e.g. controlled, sustained, extended, retarded, prolonged, delayed and the like. In embodiments in which at least one active ingredient is released in a modified manner, the modified release active ingredient is preferably contained in the core.

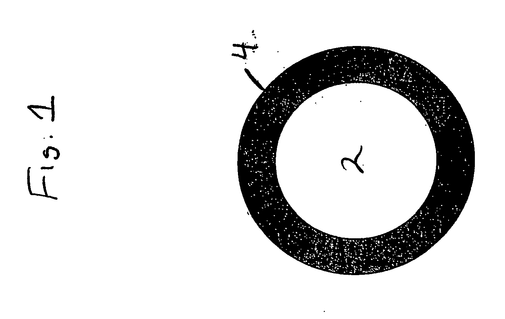

Fig. 1 depicts a cross-sectional view of one embodiment of this invention. In Fig. 1, a core 2 comprises an active ingredient. The core 2 is surrounded by a shell 4 which provides a delay of greater than one hour to the onset of dissolution of the active ingredient upon contacting of the dosage form with a liquid medium. The delay of the onset of dissolution is independent of the pH of the liquid medium. The weight of the shell material is at least 50 percent of the weight of the core material.

The core of the present invention may be prepared by any suitable method, including for example compression, or molding, and depending on the method by which it is made, typically comprises active ingredient and a variety of excipients (inactive ingredients which may be useful for conferring desired physical properties to the core).

In one embodiment, the core is prepared by the compression methods and apparatus described in copending U.S. patent application Serial No. 09/966,509, pages 16-27. Specifically, the core is made using a rotary compression module comprising a fill zone, insertion zone, compression zone, ejection zone, and purge zone in a-single apparatus having a double row die construction as shown in Figure 6 of U.S. patent application Serial No. 09/966,509. The dies of the compression module are preferably filled using the assistance of a vacuum, with filters located in or near each die. The purge zone of the compression module includes an optional powder recovery system to recover excess powder from the filters and return the powder to the dies.

In embodiments in which the core or a portion thereof is made by compression, suitable excipients include fillers, binders, disintegrants, lubricants, glidants, and the like, as known in the art. In embodiments in which the core is made by compression and additionally confers modified release of an active ingredient contained therein, the core preferably further comprises a release-modifying excipient for compression.

Suitable fillers for use in making the core, or a portion thereof, by compression include water-soluble compressible carbohydrates such as sugars, which include dextrose, sucrose, maltose, and lactose, sugar-alcohols, which include mannitol, sorbitol, maltitol, xylitol, starch hydrolysates, which include dextrins, and maltodextrins, and the like, water insoluble plastically deforming materials such as microcrystalline cellulose or other cellulosic derivatives, water-insoluble brittle fracture materials such as dicalcium phosphate, tricalcium phosphate and the like and mixtures thereof.

Suitable binders for making the core, or a portion thereof, by compression include dry binders such as polyvinyl pyrrolidone, hydroxypropylmethylcellulose, and the like; wet binders such as water-soluble polymers, including hydrocolloids such as alginates, agar, guar gum, locust bean, carrageenan, tara, gum arabic, tragacanth, pectin, xanthan, gellan, maltodextrin, galactomannan, pusstulan, laminarin, scleroglucan, gum arabic, inulin, pectin, whelan, rhamsan, zooglan, methylan, chitin, cyclodextrin, chitosan, polyvinyl pyrrolidone, cellulosics, starches, and the like; and derivatives and mixtures thereof.

Suitable disintegrants for making the core, or a portion thereof, by compression include sodium starch glycolate, cross-linked polyvinylpyrrolidone, cross-linked carboxymethylcellulose, starches, microcrystalline cellulose, and the like.

Suitable lubricants for making the core, or a portion thereof, by compression include long chain fatty acids and their salts, such as magnesium stearate and stearic acid, talc, and waxes.

Suitable glidants for making the core, or a portion thereof, by compression include colloidal silicon dioxide, and the like.

Suitable release-modifying excipients for making the core, or a portion thereof, by compression include swellable erodible hydrophilic materials, insoluble edible materials, pH-dependent polymers, and the like.

Suitable swellable erodible hydrophilic materials for use as release-modifying excipients for making the core, or a portion thereof, by compression, include water swellable cellulose derivatives, polyalkalene glycols, thermoplastic polyalkalene oxides, acrylic polymers, hydrocolloids, clays, gelling starches, and swelling cross-linked polymers, and derivitives, copolymers, and combinations thereof. Examples of suitable water swellable cellulose derivatives include sodium carboxymethylcellulose, cross-linked hydroxypropylcellulose, hydroxypropyl cellulose (HPC), hydroxypropylmethylcellulose (HPMC) such as those available from Dow Chemical Company under the tradename METHOCEL K4M, METHOCEL K15M, and METHOCEL K100M, hydroxyisopropylcellulose, hydroxybutylcellulose,hydroxyphenylcellulose, hydroxyethylcellulose (HEC), hydroxypentylcellulose, hydroxypropylethylcellulose, hydroxypropylbutylcellulose, hydroxypropylethylcellulose. Examples of suitable polyalkalene glyclols include polyethylene glycol. Examples of suitable thermoplastic polyalkalene oxides include poly (ethylene oxide). Examples of suitable acrylic polymers include potassium methacrylatedivinylbenzene copolymer, polymethylmethacrylate, CARBOPOL (high-molceular weight cross-linked acrylic acid homopolymers and copolymers), and the like. Examples of suitable hydrocolloids include alginates, agar, guar gum, locust bean gum, kappa carrageenan, iota carrageenan, tara, gum arabic, tragacanth, pectin, xanthan gum, gellan gum, maltodextrin, galactomannan, pusstulan, laminarin, scleroglucan, gum arabic, inulin, pectin, gelatin, whelan, rhamsan, zooglan, methylan, chitin, cyclodextrin, chitosan. Examples of suitable clays include smectites such as bentonite, kaolin, and laponite; magnesium trisilicate, magnesium aluminum silicate, and the like, and derivatives and mixtures thereof. Examples of suitable gelling starches include acid hydrolyzed starches, swelling starches such as sodium starch glycolate, and derivatives thereof. Examples of suitable swelling cross-linked polymers include cross-linked polyvinyl pyrrolidone, cross-linked agar, and cross-linked carboxymethylcellose sodium.

Suitable insoluble edible materials for use as release-modifying excipients for making the core, or a portion thereof, by compression include water-insoluble polymers, and low-melting hydrophobic materials. Examples of suitable water-insoluble polymers include ethylcellulose, polyvinyl alcohols, polyvinyl acetate, polycaprolactones, cellulose acetate and its derivatives, acrylates, methacrylates, acrylic acid copolymers; and the like and derivatives, copolymers, and combinations thereof. Suitable low-melting hydrophobic materials include fats, fatty acid esters, phospholipids, and waxes. Examples of suitable fats include hydrogenated vegetable oils such as for example cocoa butter, hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono, di, and triglycerides, glyceryl behenate, glyceryl palmitostearate, glyceryl monostearate, glyceryl tristearate, glyceryl trilaurylate, glyceryl myristate, GlycoWax-932, lauroyl macrogol-32 glycerides, and stearoyl macrogol-32 glycerides. Examples of suitable phospholipids include phosphotidyl choline, phosphotidyl serene, phosphotidyl enositol, and phosphotidic acid. Examples of suitable waxes include carnauba wax, spermaceti wax, beeswax, candelilla wax, shellac wax, microcrystalline wax, and paraffin wax; fat-containing mixtures such as chocolate; and the like.

Suitable pH-dependent polymers for use as release-modifying excipients for making the core, or a portion thereof, by compression include enteric cellulose derivatives, for example hydroxypropyl methylcellulose phthalate, hydroxypropyl methylcellulose acetate succinate, cellulose acetate phthalate; natural resins such as shellac and zein; enteric acetate derivatives such as for example polyvinylacetate phthalate, cellulose acetate phthalate, acetaldehyde dimethylcellulose acetate; and enteric acrylate derivatives such as for example polymethacrylate-based polymers such as poly(methacrylic acid, methyl methacrylate) 1:2, which is commercially available from Rohm Pharma GmbH under the tradename EUDRAGIT S, and poly(methacrylic acid, methyl methacrylate) 1:1, which is commercially available from Rohm Pharma GmbH under the tradename EUDRAGIT L and the like, and derivatives, salts, copolymers, and combinations thereof.

Suitable pharmaceutically acceptable adjuvants for making the core, or a portion thereof, by compression include, preservatives; high intensity sweeteners such as aspartame, acesulfame potassium, sucralose, and saccharin; flavorants; colorants; antioxidants; surfactants; wetting agents; and the like and mixtures thereof.

In certain preferred embodiments of the invention, the core, or the shell, or a portion thereof, is prepared by molding. In such embodiments, the core, or the shell, or a portion thereof, is made from a flowable material. The flowable material may be any edible material that is flowable at a temperature between about 37°C and 250°C, and that is solid, semi-solid, or can form a gel at a temperature between about -10°C and about 80°C, e.g. between about -10°C and about 55°C, or between about -10°C and about 35°C. When it is in the fluid or flowable state, the flowable material may comprise a dissolved or molten component, and optionally a solvent such as for example water or organic solvents, or combinations thereof The solvent may be partially or substantially removed by drying.

Suitable flowable materials for making the core, or the shell, or a portion thereof by molding include those comprising thermoplastic materials; film formers; thickeners such as gelling polymers or hydrocolloids; low melting hydrophobic materials; non-crystallizable carbohydrates; and the like. Suitable molten components of the flowable material include thermoplastic materials, low melting hydrophobic materials, and the like. Suitable dissolved components for the flowable material include film formers, thickeners such as gelling polymers or hydrocolloids, non-crystallizable carbohydrates, and the like.

Suitable thermoplastic materials for use as components of the flowable material for making the core or the shell or a portion thereof by molding can be molded and shaped when heated, and include both water soluble and water insoluble polymers that are generally linear, not crosslinked, nor strongly hydrogen bonded to adjacent polymer chains. Examples of suitable thermoplastic materials include thermoplastic water swellable cellulose derivatives, thermoplastic water insoluble cellulose derivatrives, thermoplastic vinyl polymers, thermoplastic starches, thermplastic polyalkalene glycols, thermoplastic polyalkalene oxides, and amorphous sugar-glass, and the like, and derivatives, copolymers, and combinations thereof. Examples of suitable thermoplastic water swellable cellulose derivatives include hydroxypropyl cellulose (HPC), hydroxypropylmethyl cellulose (HPMC), methyl cellulose (MC). Examples of suitable thermoplastic water insoluble cellulose derivatrives include cellulose acetate (CA), ethyl cellulose (EC), cellulose acetate butyrate (CAB), cellulose propionate. Examples of suitable thermoplastic vinyl polymers include, polyvinyl alcohol, polyvinyl acetate (PVA) and polyvinyl pyrrolidone (PVP). Examples of suitable thermoplastic starches are disclosed for example in U.S. Patent No. 5,427,614, which is incorporated herein by reference. Examples of suitable thermoplastic polyalkalene glycols include polyethylene glycol; Examples of suitable thermoplastic polyalkalene oxides include polyethylene oxide having a molecular weight from about 100,000 to about 900,000 Daltons. Other suitable thermoplastic materials include sugar in the form on an amorphous glass such as that used to make hard candy forms.

Any film former known in the art is suitable for use in the flowable material of the present invention. Examples of suitable film formers include, but are not limited to, film-forming water soluble polymers, film-forming proteins, film-forming water insoluble polymers, and film-forming pH-dependent polymers. Suitable film-forming water soluble polymers include water soluble vinyl polymers such as polyvinylalcohol, polyvinylacetate (PVA); water soluble polycarbohydrates such as hydroxypropyl starch, hydroxyethyl starch, pullulan, methylethyl starch, carboxymethyl starch, pre-gelatinized starches, and film-forming modified starches; water swellable cellulose derivatives such as hydroxypropyl cellulose (HPC), hydroxypropylmethyl cellulose (HPMC), methyl cellulose (MC), hydroxyethylmethylcellulose (HEMC), hydroxybutylmethylcellulose (HBMC), hydroxyethylethylcellulose (HEEC), and hydroxyethylhydroxypropylmethyl cellulose (HEMPMC); water soluble copolymers such as methacrylic acid and methacrylate ester copolymers, polyvinyl alcohol and polyethylene glycol copolymers, polyethylene oxide and polyvinylpyrrolidone copolymers; and derivatives and combinations thereof. Suitable film-forming proteins may be natural or chemically modified, and include gelatin, whey protein, myofibrillar proteins, coaggulatable proteins such as albumin, casein, caseinates and casein isolates, soy protein and soy protein isolates, zein;; and polymers, derivatives and mixtures thereof. Suitable film-forming water insoluble polymers, include for example ethylcellulose, polyvinyl alcohols, polyvinyl acetate, polycaprolactones, cellulose acetate and its derivatives, acrylates, methacrylates, acrylic acid copolymers; and the like and derivatives, copolymers, and combinations thereof. Suitable film-forming pH-dependent polymers include enteric cellulose derivatives, such as for example hydroxypropyl methylcellulose phthalate, hydroxypropyl methylcellulose acetate succinate, cellulose acetate phthalate; natural resins such as shellac and zein; enteric acetate derivatives such as for example polyvinylacetate phthalate, cellulose acetate phthalate, acetaldehyde dimethylcellulose acetate; and enteric acrylate derivatives such as for example polymethacrylate-based polymers such as poly(methacrylic acid, methyl methacrylate) 1:2, which is commercially available from Rohm Pharma GmbH under the tradename EUDRAGIT S and poly(methacrylic acid, methyl methacrylate) 1:1, which is commercially available from Rohm Pharma GmbH under the tradename EUDRAGIT L and the like, and derivatives, salts, copolymers, and combinations thereof.

One suitable hydroxypropylmethylcellulose compound for use as a thermoplastic film-forming water soluble polymer is "HPMC 2910", which is a cellulose ether having a degree of substitution of about 1.9 and a hydroxypropyl molar substitution of 0.23, and containing, based upon the total weight of the compound, from about 29% to about 30% methoxyl groups and from about 7% to about 12% hydroxylpropyl groups. HPMC 2910 is commercially available from the Dow Chemical Company under the tradename METHOCEL E. METHOCEL E5, which is one grade of HPMC-2910 suitable for use in the present invention, has a viscosity of about 4 to 6 cps (4 to 6 millipascal-seconds) at 20°C in a 2% aqueous solution as determined by a Ubbelohde viscometer. Similarly, METHOCEL E6, which is another grade of HPMC-2910 suitable for use in the present invention, has a viscosity of about 5 to 7 cps (5 to 7 millipascal-seconds) at 20°C in a 2% aqueous solution as determined by a Ubbelohde viscometer. METHOCEL E15, which is another grade of HPMC-2910 suitable for use in the present invention, has a viscosity of about 15000 cps (15 millipascal-seconds) at 20°C in a 2% aqueous solution as determined by a Ubbelohde viscometer. As used herein, "degree of substitution" shall mean the average number of substituent groups attached to a anhydroglucose ring, and "hydroxypropyl molar substitution" shall mean the number of moles of hydroxypropyl per mole anhydroglucose.

One suitable polyvinyl alcohol and polyethylene glycol copolymer is commercially available from BASF Corporation under the tradename KOLLICOAT IR.

As used herein, "modified starches" include starches that have been modified by crosslinking, chemically modified for improved stability or optimized performance, or physically modified for improved solubility properties or optimized performance. Examples of chemically-modified starches are well known in the art and typically include those starches that have been chemically treated to cause replacement of some of its hydroxyl groups with either ester or ether groups. Crosslinking, as used herein, may occur in modified starches when two hydroxyl groups on neighboring starch molecules are chemically linked. As used herein, "pre-gelatinized starches" or "instantized starches" refers to modified starches that have been pre-wetted, then dried to enhance their cold-water solubility. Suitable modified starches are commercially available from several suppliers such as, for example, A.E. Staley Manufacturing Company, and National Starch & Chemical Company. One suitable film forming modified starch includes the pre-gelatinized waxy maize derivative starches that are commercially available from National Starch & Chemical Company under the tradenames PURITY GUM and FILMSET, and derivatives, copolymers, and mixtures thereof. Such waxy maize starches typically contain, based upon the total weight of the starch, from about 0 percent to about 18 percent of amylose and from about 100% to about 88% of amylopectin.

Another suitable film forming modified starch includes the hydroxypropylated starches, in which some of the hydroxyl groups of the starch have been etherified with hydroxypropyl groups, usually via treatment with propylene oxide. One example of a suitable hydroxypropyl starch that possesses film-forming properties is available from Grain Processing Company under the tradename, PURE-COTE B790.

Suitable tapioca dextrins for use as film formers include those available from National Starch & Chemical Company under the tradenames CRYSTAL GUM or K-4484, and derivatives thereof such as modified food starch derived from tapioca, which is available from National Starch and Chemical under the tradename PURITY GUM 40, and copolymers and mixtures thereof.

Any thickener known in the art is suitable for use in the flowable material. Examples of such thickeners include but are not limited to hydrocolloids (also referred to herein as gelling polymers), clays, gelling starches, and crystallizable carbohydrates, and derivatives, copolymers and mixtures thereof. Examples of suitable hydrocolloids (also referred to herein as gelling polymers) such as alginates, agar, guar gum, locust bean, carrageenan, tara, gum arabic, tragacanth, pectin, xanthan, gellan, maltodextrin, galactomannan, pusstulan, laminarin, scleroglucan, gum arabic, inulin, pectin, whelan, rhamsan, zooglan, methylan, chitin, cyclodextrin, chitosan. Examples of suitable clays include smectites such as bentonite, kaolin, and laponite; magnesium trisilicate, magnesium aluminum silicate, and the like, and derivatives and mixtures thereof. Examples of suitable gelling starches include acid hydrolyzed starches, and derivatives and mixtures thereof. Additional suitable thickening hydrocolloids include low-moisture polymer solutions such as mixtures of gelatin and other hydrocolloids at water contents up to about 30%, such as for example those used to make "gummi" confection forms. Suitable crystallizable carbohydrates include the monosaccharides and the oligosaccharides. Of the monosaccharides, the aldohexoses e.g., the D and L isomers of allose, altrose, glucose, mannose, gulose, idose, galactose, talose, and the ketohexoses e.g., the D and L isomers of fructose and sorbose along with their hydrogenated analogs: e.g., glucitol (sorbitol), and mannitol are preferred. Of the oligosaccharides, the 1,2-disaccharides sucrose and trehalose, the 1,4-disaccharides maltose, lactose, and cellobiose, and the 1,6-disaccharides gentiobiose and melibiose, as well as the trisaccharide raffinose are preferred along with the isomerized form of sucrose known as isomaltulose and its hydrogenated analog isomalt. Other hydrogenated forms of reducing disaccharides (such as maltose and lactose), for example, maltitol and lactitol are also preferred. Additionally, the hydrogenated forms of the aldopentoses: e.g., D and L ribose, arabinose, xylose, and lyxose and the hydrogenated forms of the aldotetroses: e.g., D and L erythrose and threose are preferred and are exemplified by xylitol and erythritol, respectively.

In one embodiment of the invention, the flowable material comprises gelatin as a gelling polymer. Gelatin is a natural, thermogelling polymer. It is a tasteless and colorless mixture of derived proteins of the albuminous class which is ordinarily soluble in warm water. Two types of gelatin - Type A and Type B - are commonly used. Type A gelatin is a derivative of acid-treated raw materials. Type B gelatin is a derivative of alkali-treated raw materials. The moisture content of gelatin, as well as its Bloom strength, composition and original gelatin processing conditions, determine its transition temperature between liquid and solid. Bloom is a standard measure of the strength of a gelatin gel, and is roughly correlated with molecular weight. Bloom is defined as the weight in grams required to move a half-inch diameter plastic plunger 4 mm into a 6.67% gelatin gel that has been held at 10°C for 17 hours. In a preferred embodiment, the flowable material is an aqueous solution comprising 20% 275 Bloom pork skin gelatin, 20% 250 Bloom Bone Gelatin, and approximately 60% water.

Suitable xanthan gums include those available from C.P. Kelco Company under the tradenames KELTROL 1000, XANTROL 180, or K9B310.

"Acid-hydrolyzed starch," as used herein, is one type of modified starch that results from treating a starch suspension with dilute acid at a temperature below the gelatinization point of the starch. During the acid hydrolysis, the granular form of the starch is maintained in the starch suspension, and the hydrolysis reaction is ended by neutralization, filtration and drying once the desired degree of hydrolysis is reached. As a result, the average molecular size of the starch polymers is reduced. Acid-hydrolyzed starches (also known as "thin boiling starches") tend to have a much lower hot viscosity than the same native starch as well as a strong tendency to gel when cooled.

"Gelling starches," as used herein, include those starches that, when combined with water and heated to a temperature sufficient to form a solution, thereafter form a gel upon cooling to a temperature below the gelation point of the starch. Examples of gelling starches include, but are not limited to, acid hydrolyzed starches such as that available from Grain Processing Corporation under the tradename PURE-SET B950; hydroxypropyl distarch phosphate such as that available from Grain Processing Corporation under the tradename, PURE-GEL B990, and mixtures thereof.

Suitable low-melting hydrophobic materials for use as components of the flowable material for making the core, or the shell, or a portion thereof by molding include fats, fatty acid esters, phospholipids, and waxes. Examples of suitable fats include hydrogenated vegetable oils such as for example cocoa butter, hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono, di, and triglycerides, glyceryl behenate, glyceryl palinitostearate, glyceryl monostearate, glyceryl tristearate, glyceryl trilaurylate, glyceryl myristate, GlycoWax-932, lauroyl macrogol-32 glycerides, and stearoyl macrogol-32 glycerides. Examples of suitable phospholipids include phosphotidyl choline, phosphotidyl serene, phosphotidyl enositol, and phosphotidic acid. Examples of suitable waxes include carnauba wax, spermaceti wax, beeswax, candelilla wax, shellac wax, microcrystalline wax, and paraffin wax; fat-containing mixtures such as chocolate; and the like.

Suitable non-crystallizable carbohydrates for use as components of the flowable material for making the core, or the shell, or a portion thereof by molding include non-crystallizable sugars such as polydextrose, and starch hydrolysates, e.g. glucose syrup, corn syrup, and high fructose corn syrup; and non-crystallizable sugar-alcohols such as maltitol syrup.

Suitable solvents for optional use as components of the flowable material for making the core, or the shell, or a portion thereof by molding include water; polar organic solvents such as methanol, ethanol, isopropanol, acetone, and the like; and non-polar organic solvents such as methylene chloride, and the like; and mixtures thereof.

The flowable material for making the core or the shell or a portion thereof by molding may optionally comprise adjuvants or excipients, which may comprise up to about 30% by weight of the flowable material. Examples of suitable adjuvants or excipients include plasticizers, detackifiers, humectants, surfactants, anti-foaming agents, colorants, flavorants, sweeteners, opacifiers, and the like. Suitable plasticizers for making the core, the shell, or a portion thereof, by molding include, but not be limited to polyethylene glycol; propylene glycol; glycerin; sorbitol; triethyl citrate; tribuyl citrate; dibutyl sebecate; vegetable oils such as castor oil, rape oil, olive oil, and sesame oil; surfactants such as polysorbates, sodium lauryl sulfates, and dioctyl-sodium sulfosuccinates; mono acetate of glycerol; diacetate of glycerol; triacetate of glycerol; natural gums; triacetin; acetyltributyl citrate; diethyloxalate; diethylmalate; diethyl fumarate; diethylmalonate; dioctylphthalate; dibutylsuccinate; glyceroltributyrate; hydrogenated castor oil; fatty acids; substituted triglycerides and glycerides; and the like and/or mixtures thereof. In certain embodiments, the shell is substantially free of plasticizers, i.e. contains less than about 1%, say less than about 0.01 % of plasticizers.

In one preferred embodiment, the flowable material comprises less than 5% humectants, or alternately is substantially free of humectants, such as glycerin, sorbitol, maltitol, xylitol, or propylene glycol. Humectants have traditionally been included in pre-formed films employed in enrobing processes, such as that disclosed in U.S. Patent Nos. 5,146,730 and 5,459,983, assigned to Banner Gelatin Products Corp., in order to ensure adequate flexibility or plasticity and bondability of the film during processing. Humectants function by binding water and retaining it in the film. Pre-formed films used in enrobing processes can typically comprise up to 45% water. Disadvantageously, the presence of humectant prolongs the drying process, and can adversely affect the stability of the finished dosage form.

In one embodiment of the invention, the core, the shell, or a portion thereof is made by the thermal setting molding method and apparatus described in copending U.S. patent application Serial No. 09/966,450, pages 57-63. In this embodiment, the core, the shell, or a portion thereof is formed by injecting a starting material in flowable form into a molding chamber. The starting material preferably comprises an active ingredient and a thermal setting material at a temperature above the melting point of the thermal setting material but below the decomposition temperature of the active ingredient. The starting material is cooled and solidifies in the molding chamber into a shaped form (i.e., having the shape of the mold).

According to this method, the starting material must be in flowable form. For example, it may comprise solid particles suspended in a molten matrix, for example a polymer matrix. The starting material may be completely molten or in the form of a paste. The starting material may comprise an active ingredient dissolved in a molten material. Alternatively, the starting material may be made by dissolving a solid in a solvent, which solvent is then evaporated from the starting material after it has been molded.

The starting material may comprise any edible material which is desirable to incorporate into a shaped form, including active ingredients, nutritionals, vitamins, minerals, flavors, sweeteners, and the like. Preferably, the starting material comprises an active ingredient and a thermal setting material. The thermal setting material may be any edible material that is flowable at a temperature between about 37 and about 120°C, and that is a solid at a temperature between about 0 and about 35°C. Preferred thermal setting materials include water-soluble polymers such as polyalkylene glycols, polyethylene oxides and derivatives, and sucrose esters; fats such as cocoa butter, hydrogenated vegetable oil such as palm kernel oil, cottonseed oil, sunflower oil, and soybean oil; mono-, di-, and triglycerides, phospholipids, waxes such as carnuba wax, spermaceti wax, beeswax, candelilla wax, shellac wax, microcrystalline wax, and paraffin wax; fat-containing mixtures such as chocolate; sugar in the form on an amorphous glass such as that used to make hard candy forms, sugar in a supersaturated solution such as that used to make fondant forms; low-moisture polymer solutions such as mixtures of gelatin and other hydrocolloids at water contents up to about 30% such as those used to make "gummi" confection forms. In a particularly preferred embodiment, the thermal setting material is a water-soluble polymer such as polyethylene glycol.

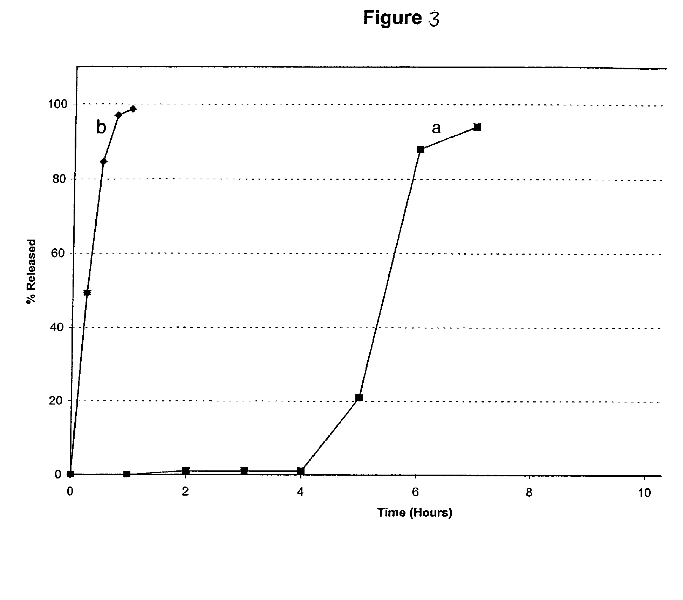

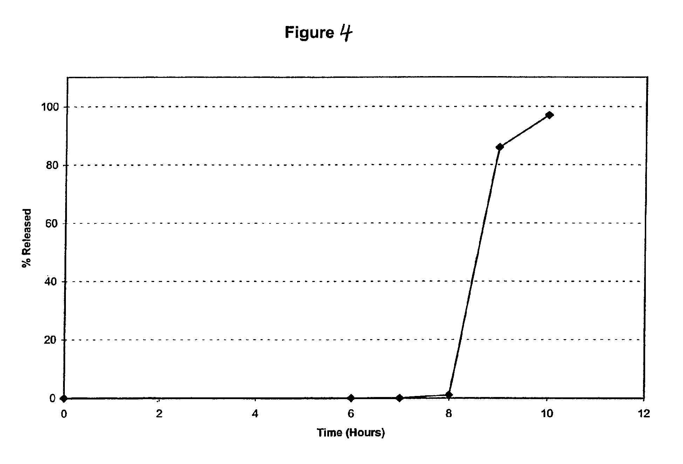

In another embodiment of the invention, the core, the shell, or a portion thereof is make using the thermal cycle molding method and apparatus described in copending U.S. patent application Serial No. 09/966,497, pages 27-51. In the thermal cycle molding method and apparatus of U.S. patent application Serial No. 09/966,497, a thermal cycle molding module having the general configuration shown in Figure 3 therein is employed. The thermal cycle molding module 200 comprises a rotor 202 around which a plurality of mold units 204 are disposed. The thermal cycle molding module includes a reservoir 206 (see Figure 4) for holding flowable material to make the core. In addition, the thermal cycle molding module is provided with a temperature control system for rapidly heating and cooling the mold units. Figures 55 and 56 depict such a temperature control system 600.

In certain embodiments the core or portions thereof may be molded using a solvent-free process. In such embodiments, the core may comprise active ingredient contained within an excipient matrix. The matrix typically comprises at least about 30 percent, e.g. at least about 45 weight percent of a thermal-reversible carrier, and optionally up to about 30 weight percent of various adjuvants such as for example plasticizers, gelling agents, strengthening agents, colorants, stabilizers, preservatives, and the like as known in the art. The matrix may optionally further comprise up to about 55 weight percent of one or more release-modifying moldable excipients as described below.

The core may be in a variety of different shapes. For example, the core may be shaped as a polyhedron, such as a cube, pyramid, prism, or the like; or may have the geometry of a space figure with some non-flat faces, such as a cone, truncated cone, cylinder, sphere, torus, or the like. In cetrain embodiments, the core has one or more major faces. For example in embodiments wherein the core is a compressed tablet, the core surface typically has two opposing major faces formed by contact with the upper and lower punch faces in the compression machine. In such embodiments the core surface typically further comprises a "belly-band" located between the two major faces, and formed by contact with the die walls in the compression machine. Exemplary core shapes which may be employed include tablet shapes formed from compression tooling shapes described by "The Elizabeth Companies Tablet Design Training Manual," (Elizabeth Carbide Die Co., Inc., p.7 (McKeesport, Pa.)) as follows (the tablet shape corresponds inversely to the shape of the compression tooling):

1. Shallow Concave.
2. Standard Concave.
3. Deep Concave.
4. Extra Deep Concave.
5. Modified Ball Concave.
6. Standard Concave Bisect.
7. Standard Concave Double Bisect.
8. Standard Concave European Bisect.
9. Standard Concave Partial Bisect.
10. Double Radius.
11. Bevel & Concave.
12. Flat Plain.
13. Flat-Faced-Beveled Edge (F.F.B.E.).
14. F.F.B.E. Bisect.
15. F.F.B.E. Double Bisect.
16. Ring.
17. Dimple.
18. Ellipse.
19. Oval.
20. Capsule.
21. Rectangle.
22. Square.
23. Triangle.
24. Hexagon.
25. Pentagon.
26. Octagon.
27. Diamond.
28. Arrowhead.
29. Bullet.
30. Barrel.
31. Half Moon.
32. Shield.
33. Heart.
34. Almond.
35. House/Home Plate.
36. Parallelogram.
37. Trapezoid.
38. Figure 8/Bar Bell.
39. Bow Tie.
40. Uneven Triangle.

In one embodiment of the invention, the core comprises multiple portions, for example a first portion and a second portion. The portions may be prepared by the same or different methods and mated using various techniques, such as thermal cycle molding and thermal setting molding methods described herein. For example, the first and second portions may both be made by compression, or both may be made by molding. Or one portion may be made by compression and the other by molding. The same or different active ingredient may be present in the first and second portions of the core. Alternately, one or more core portions may be substantially free of active ingredients.

In certain embodiments of the invention, the core or a portion thereof may function to confer modified release properties to at least one active ingredient contained therein. In such embodiments, wherein the core or core portion is made by compression, as previously noted, the core preferably comprises a release-modifying excipient for compression. In such embodiments, wherein the core or core portion is made by molding, as previously noted, the core preferably comprises a release-modifying moldable excipient.

In embodiments in which the core or a portion thereof functions as an eroding matrix from which dispersed active ingredient is liberated in a sustained, extended, prolonged, or retarded manner, the core portion preferably comprises a release-modifying compressible or moldable excipient selected from swellable erodible hydrophilic materials, pH-dependent polymers, and combinations thereof.

In embodiments in which the core or a portion thereof functions as a diffusional matrix through which active ingredient is liberated in a sustained, extended, prolonged, or retarded manner, the core portion preferably comprises a release-modifying excipient selected from combinations of insoluble edible materials and pore-formers. Alternately, in such embodiments in which the core portion is prepared by molding, the thermal-reversible carrier may function by dissolving and forming pores or channels through which the active ingredient may be liberated.

The shell of the present invention may be prepared by molding, using a solvent-free process, and depending on the method by which it is made, typically comprises a variety of excipients which are useful for conferring desired properties to the shell. The shell may optionally further comprise one or more active ingredients.

The shell comprises at least 30 percent, e.g. at least about 45 percent by weight of the thermal-reversible carrier. The shell may optionally further comprise up to about 55 weight percent of a release modifying excipient. The shell may optionally further comprise up to about 30 weight percent total of various plasticizers, adjuvants and excipients. In certain embodiments in which the shell is prepared by solvent-free molding, the release modifying excipient is preferrably selected from swellable, erodible hydrophilic materials.

Suitable thermal-reversible carriers for making the core, or a portion thereof, by solvent-free molding are thermoplastic materials typically having a melting point below about 110°C, more preferably between about 20 and about 100°C. Examples of suitable thermal-reversible carriers for solvent-free molding include thermplastic polyalkalene glycols, thermoplastic polyalkalene oxides, low melting hydrophobic materials, thermoplastic polymers, thermoplastic starches, and the like. Suitable thermoplastic polyalkylene glycols for use as thermal-reversible carriers include polyethylene glycol having molecular weight from about 100 to about 20,000, e.g. from about 100 to about 8,000, say from about 1000 to about 8,000 Daltons. Suitable thermoplastic polyalkalene oxides include polyethylene oxide having a molecular weight from about 100,000 to about 900,000 Daltons. Suitable low-melting hydrophobic materials for use as thermal-reversible carriers include fats, fatty acid esters, phospholipids, and waxes which are solid at room temperature, fat-containing mixtures such as chocolate; and the like. Examples of suitable fats include hydrogenated vegetable oils such as for example cocoa butter, hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono, di, and triglycerides, glyceryl behenate, glyceryl palmitostearate, glyceryl monostearate, glyceryl tristearate, glyceryl trilaurylate, glyceryl myristate, GlycoWax-932, lauroyl macrogol-32 glycerides, and stearoyl macrogol-32 glycerides. Examples of suitable phospholipids include phosphotidyl choline, phosphotidyl serene, phosphotidyl enositol, and phosphotidic acid. Examples of suitable waxes that are solid at room temperature include carnauba wax, spermaceti wax, beeswax, candelilla wax, shellac wax, microcrystalline wax, and paraffin wax. Suitable thermoplastic polymers for use as thermal-reversible carriers include thermoplastic water swellable cellulose derivatives, thermoplastic water insoluble polymers, thermoplastic vinyl polymers. Suitable thermoplastic water swellable cellulose derivatives include include hydroxypropylmethyl cellulose (HPMC), methyl cellulose (MC), carboxymethylcellulose (CMC), cross-linked hydroxypropylcellulose, hydroxypropyl cellulose (HPC), hydroxybutylcellulose (HBC), hydroxyethylcellulose (HEC), hydroxypropylethylcellulose, hydroxypropylbutylcellulose, hydroxypropylethylcellulose, and salts, derivatives, copolymers, and combinations thereof. Suitable thermoplastic water insoluble polymers include ethylcellulose, polyvinyl alcohols, polyvinyl acetate, polycaprolactones, cellulose acetate and its derivatives, acrylates, methacrylates, acrylic acid copolymers, and the like and derivatives, copolymers, and combinations thereof. Suitable thermoplastic vinyl polymer's include polyvinylacetate, polyvinyl alcohol, and polyvinyl pyrrolidone (PVP). Examples of suitable thermoplastic starches for use as thermal-reversible carriers include those disclosed in U.S. Patent No. 5,427,614, which is incorporated herein by reference.

In one embodiment, the thermal-reversible carrier for making the core, or a portion thereof, by solvent-free molding is selected from polyalkylene glycols, polyalkaline oxides, and combinations thereof. In one particular such embodiment, the core or shell or portion thereof further comprises shellac as a strengthening adjuvant. Advantageously, shellac may be employed as a strengthening agent in the molded core or shell or portions thereof at a level from about 5 to about 15 weight percent of the molded portion, without imparting pH-dependency to the dissolution of the molded portion.

Suitable release-modifying moldable excipients for making the core, or a portion thereof, by solvent-free or sovent based molding include but are not limited to swellable erodible hydrophilic materials, pH-dependent polymers, pore formers, and insoluble edible materials.

In embodiments of the present invention wherein the shell is prepared by a solvent-free or solvent-based molding process, suitable release-modifying excipients are preferably selected from swellable erodible hydrophilic materials, pH dependent polymers, and insoluble edible materials.

Suitable swellable erodible hydrophilic materials for use as release-modifying excipients for making the core,

or the shell, or a portion thereof by a solvent-free molding process include water swellable cellulose derivatives, polyalkalene glycols, thermoplastic polyalkalene oxides, acrylic polymers, hydrocolloids, clays, gelling starches, and swelling cross-linked polymers, and derivitives, copolymers, and combinations thereof. Examples of suitable water swellable cellulose derivatives include sodium carboxymethylcellulose, cross-linked hydroxypropylcellulose, hydroxypropyl cellulose (HPC), hydroxypropylmethylcellulose (HPMC), hydroxyisopropylcellulose, hydroxybutylcellulose,hydroxyphenylcellulose, hydroxyethylcellulose (HEC), hydroxypentylcellulose, hydroxypropylethylcellulose, hydroxypropylbutylcellulose, hydroxypropylethylcellulose. Examples of suitable polyalkalene glyclols include polyethylene glycol. Examples of suitable thermoplastic polyalkalene oxides include poly (ethylene oxide). Examples of suitable acrylic polymers include potassium methacrylatedivinylbenzene copolymer, polymethylmethacrylate, CARBOPOL (high-molceular weight cross-linked acrylic acid homopolymers and copolymers), and the like. Examples of suitable hydrocolloids include alginates, agar, guar gum, locust bean gum, kappa carrageenan, iota carrageenan, tara, gum arabic, tragacanth, pectin, xanthan gum, gellan gum, maltodextrin, galactomannan, pusstulan, laminarin, scleroglucan, gum arabic, inulin, pectin, gelatin, whelan, rhamsan, zooglan, methylan, chitin, cyclodextrin, chitosan. Examples of suitable clays include smectites such as bentonite, kaolin, and laponite; magnesium trisilicate, magnesium aluminum silicate, and the like, and derivatives and mixtures thereof. Examples of suitable gelling starches include acid hydrolyzed starches, swelling starches such as sodium starch glycolate, and derivatives thereof. Examples of suitable swelling cross-linked polymers include cross-linked polyvinyl pyrrolidone, cross-linked agar, and cross-linked carboxymethylcellose sodium.

Suitable pH-dependent polymers for use as release-modifying moldable excipients for making the molded matrix or molded core or molded shell or a portion thereof by molding include enteric cellulose derivatives, for example hydroxypropyl methylcellulose phthalate, hydroxypropyl methylcellulose acetate succinate, cellulose acetate phthalate; natural resins such as shellac and zein; enteric acetate derivatives such as for example polyvinylacetate phthalate, cellulose acetate phthalate, acetaldehyde dimethylcellulose acetate; and enteric acrylate derivatives such as for example polymethacrylate-based polymers such as poly(methacrylic acid, methyl methacrylate) 1:2, which is commercially available from Rohm Pharma GmbH under the tradename EUDRAGIT S and poly(methacrylic acid, methyl methacrylate) 1:1, which is commercially available from Rohm Pharma GmbH under the tradename EUDRAGIT L and the like, and derivatives, salts, copolymers, and combinations thereof.

Suitable pore formers for use as release-modifying excipients for making the molded matrix, the core, the shell, or a portion thereof by molding include water-soluble organic and inorganic materials. Examples of suitable water-soluble organic materials include water soluble polymers including water soluble cellulose derivatives such as hydroxypropylmethylcellulose, and hydroxypropylcellulose; water soluble carbohydrates such as sugars, and starches; water soluble polymers such as polyvinylpyrrolidone and polyethylene glycol, and insoluble swelling polymers such as microcrystalline cellulose. Examples of suitable water soluble inorganic materials include salts such as sodium chloride and potassium chloride and the like and/or mixtures thereof.

Suitable insoluble edible materials for use as release-modifying moldable excipients for making the core, or the shell, or a portion thereof, by molding, include water-insoluble polymers, and low-melting hydrophobic materials. Examples of suitable water-insoluble polymers include ethylcellulose, polyvinyl alcohols, polyvinyl acetate, polycaprolactones, cellulose acetate and its derivatives, acrylates, methacrylates, acrylic acid copolymers; and the like and derivatives, copolymers, and combinations thereof. Suitable low-melting hydrophobic materials include fats, fatty acid esters, phospholipids, and waxes. Examples of suitable fats include hydrogenated vegetable oils such as for example cocoa butter, hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono, di, and triglycerides, glyceryl behenate, glyceryl palmitostearate, glyceryl monostearate, glyceryl tristearate, glyceryl trilaurylate, glyceryl myristate, GlycoWax-932, lauroyl macrogol-32 glycerides, and stearoyl macrogol-32 glycerides. Examples of suitable phospholipids include phosphotidyl choline, phosphotidyl serene, phosphotidyl enositol, and phosphotidic acid. Examples of suitable waxes include carnauba wax, spermaceti wax, beeswax, candelilla wax, shellac wax, microcrystalline wax, and paraffin wax; fat-containing mixtures such as chocolate; and the like.

The shell of the present invention, whether prepared by a solvent-free molding process, or by a solvent-based molding process, is substantially free of pores having a diameter of 0.5-5.0 micrometres. As used herein, "substantially free" means that the shell has a pore volume of less than about 0.02 $cm^3/g$, preferably less than about 0.01 $cm^3/g$, more preferably less than about 0.005 $cm^3/g$ in the pore diameter range of 0.5 to 5.0 micrometres. In contrast, typical compressed materials have pore volumes of more than about 0.02 $cm^3/g$ in this diameter range.

The pore volume, pore diameter and density of the shells used in this invention may be determined using a Quantachrome Instruments PoreMaster 60 mercury intrusion porosimeter and associated computer software program known as "Porowin." The procedure is documented in the Quantachrome Instruments PoreMaster Operation Manual. The PoreMaster determines both pore volume and pore diameter of a solid or powder by forced intrusion of a non-wetting liquid (mercury), which involves evacuation of the sample in a sample cell (penetrometer), filling the cell with mercury to surround the sample with mercury, applying pressure to the sample cell by: (i) compressed air (up to 345 kPa [50 psi] maximum); and (ii) a hydraulic (oil) pressure generator (up to 414 MPa [60000 psi] maximiun). Intruded volume is measured by a change in the capacitance as mercury moves from outside the sample into its pores under applied pressure. The corresponding pore size diameter (d) at which the intrusion takes place is calculated directly from the so-called "Washbum Equation": d= - (4γ(cosθ))/P where γ is the surface tension of liquid mercury, θ is the contact angle between mercury and the sample surface and P is the applied pressure.

Equipment used for pore volume measurements:

(1) Quantachrome Instruments PoreMaster 60.
(2) Analytical Balance capable of weighing to 0.0001g.
(3) Desiccator.

Reagents used for measurements:

(1) High purity nitrogen.
(2) Triply distilled mercury.
(3) High pressure fluid (Dila AX, available from Shell Chemical Co.).
(4) Liquid nitrogen (for Hg vapor cold trap).
(5) Isopropanol or methanol for cleaning sample cells.
(6) Liquid detergent for cell cleaning.

Procedure:

The samples remain in sealed packages or as received in the dessicator until analysis. The vacuum pump is switched on, the mercury vapor cold trap is filled with liquid nitrogen, the compressed gas supply is regulated at 379 kPa [55 psi], and the instrument is turned on and allowed a warm up time of at least 30 minutes. The empty penetrometer cell is assembled as described in the instrument manual and its weight is recorded. The cell is installed in the low pressure station and "evacuation and fill only" is selected from the analysis menu, and the following settings are employed:

Fine Evacuation time: 1 min.
Fine Evacuation rate: 10
Coarse Evacuation time: 5 min.

The cell (filled with mercury) is then removed and weighed. The cell is then emptied into the mercury reservoir, and two tablets from each sample are placed in the cell and the cell is reassembled. The weight of the cell and sample are then recorded. The cell is then installed in the low-pressure station, the low-pressure option is selected from the menu, and the following parameters are set:

Mode: Low pressure
Fine evacuation rate: 10
Fine evacuation until: 200 micrometre Hg
Coarse evacuation time: 10 min.
Fill pressure: Contact +0.1
Maximum pressure: 50
Direction: Intrusion And Extrusion
Repeat: 0
Mercury contact angle; 140
Mercury surface tension: 480

Data acquisition is then begun. The pressure vs. cumulative volume-intruded plot is displayed on the screen. After low-pressure analysis is complete, the cell is removed from the low-pressure station and reweighed. The space above the mercury is filled with hydraulic oil, and the cell is assembled and installed in the high-pressure cavity. The following settings are used:

Mode: Fixed rate
Motor speed: 5
Start pressure: 20
End pressure: 60,000
Direction: Intrusion and extrusion
Repeat: 0

Oil fill length: 5
Mercury contact angle: 140
Mercury surface tension: 480

Data acquisition is then begun and graphic plot pressure vs. intruded volume is displayed on the screen. After the high pressure run is complete, the low-and high-pressure data files of the same sample are merged.

The shell of the present invention, whether prepared by a solvent-free molding process, or by a solvent-based molding process, typically has a surface gloss of at least about 150 gloss units, e.g. at least about 175 gloss units, or at least about 190 gloss units, when measured according to the method set forth in Example 6 herein. In contrast, typical sprayed coatings have gloss values of less than about 150 gloss units.

Dosage forms with high surface gloss are preferred by consumers due to their aesthetic elegance and perceived swallowability. The surface gloss of the shell depends upon a number of factors, including the shell composition, the method of forming the shell, and, if a mold is used, the surface finish on the mold.

Shells of this invention may be tested for surface gloss using an instrument available from TriCor Systems Inc. (Elgin, IL) under the tradename TRI-COR MODEL 805A/806H SURFACE ANALYSIS SYSTEM and generally in accordance with the procedure described in "TriCor Systems WGLOSS 3.4 Model 805A/806H Surface Analysis System Reference Manual" (1996), which is incorporated by reference herein, except as modified below.

This instrument utilizes a CCD camera detector, employs a flat diffuse light source, compares samples to a reference standard, and determines average gloss values at a 60 degree incident angle. During its operation, the instrument generates a gray-scale image, wherein the occurrence of brighter pixels indicates the presence of more gloss at that given location.

The instrument also incorporates software that utilizes a grouping method to quantify gloss, i.e., pixels with similar brightness are grouped together for averaging purposes.

The "percent full scale" or "percent ideal" setting (also referred to as the "percent sample group" setting), is specified by the user to designate the portion of the brightest pixels above the threshold that will be considered as one group and averaged within that group. "Threshold," as used herein, is defined as the maximum gloss value that will not be included in the average gloss value calculation. Thus, the background, or the non-glossy areas of a sample are excluded from the average gloss value calculations. The method disclosed in K. Fegley and C. Vesey, "The Effect of Tablet Shape on the Perception of High Gloss Film Coating Systems," which is available at www.colorcon.com as of 18 March, 2002, is used to minimize the effects resulting from different shell shapes, and to report a metric that is comparable across the industry. (The 50% sample group setting is selected as the setting which best approximates analogous data from surface roughness measurements.)

After initially calibrating the instrument using a calibration reference plate (190-228; 294 degree standard; no mask, rotation 0, depth 0), a standard surface gloss measurement may be created using gel-coated caplets available from McNeil-PPC, Inc. under the tradename Extra Strength TYLENOL Gelcaps. The average gloss value for a sample of such gel-coated caplets may be determined, while employing the 25 mm full view mask (190-280), and configuring the instrument to the following settings:

Rotation: 0
Depth: 0.635cm (0.25 inches)
Gloss Threshold: 95
% Full Scale: 50%
Index of Refraction: 1.57

The average surface gloss value for the reference standard is then determined.

Each shell sample may then be independently tested in accordance with the same procedure.
In embodiments wherein the shell is prepared by a solvent-free molding process, the weight of the shell is typically from about 50 to about 400%, e.g. from about 75 to about 400%, or about 100 to about 200% percent of the weight of the core. In embodiments wherein the shell is prepared by a solvent-based molding process, the weight of the shell is typically from about 10 to about 100 percent, preferably from about 10 to about 60 percent of the weight of the core.

The shell provides a delay of greater than one hour, for example at least about 3 hours, or at least about 4 hours, or at least about 6 hours, or at least about 12 hours to the onset of dissolution of the active ingredient upon contacting of the dosage form with a liquid medium such as water, gastrointestinal fluid or the like. The delay period is typically controlled by the shell thickness, composition, or a combination thereof. In one embodiment the delay period is independent of the pH of the dissolution media or fluid environment. For example, the average lag-time for dissolution of active ingredient in 0.1 N HCl is not substantially different (i.e. within about 30 minutes, preferably within about 15 minutes) from the average lag-time for the dissolution of active ingredient in pH 5.6 buffer system.

Shell thicknesses which may be employed in this invention are 500 to 4000 micrometres. In embodiments wherein the shell is prepared by a solvent-free molding process, the shell typically has a thickness of about 500 to about 2000 micrometres, say about 800 to about 1200 micrometres.

In one embodiment of the invention, the shell additionally comprises at least one active ingredient which may be the same or different than the active ingredient contained in the core.

In another embodiment of this invention, at least one active ingredient contained within the dosage form exhibits a delayed burst release profile. By "delayed burst release profile" it is meant that the release of that particular active ingredient from the dosage form is delayed for a pre-determined time after ingestion by the patient, and the delay period ("lag time") is followed by prompt (immediate) release of that active ingredient. The shell of the present invention provides for the delay period and is preferaby substantially free of the active ingredient to be released in a delayed burst manner. In such embodiments, the delayed burst active ingredient is typically contained within the core or a portion thereof. In these embodiments, the core may be prepared by compression or molding, and is formulated for immediate release, as is known in the art, so that the core is readily soluble upon contact with the dissolution medium. In such embodiments the core preferably comprises a disintegrant, and optionally comprises additional excipients such as fillers or thermoplastic materials selected from water-soluble or low-melting materials, and surfactants or wetting agents. In these embodiments, the dissolution of the burst release active ingredient, after the delay period, meets USP specifications for immediate release tablets containing that active ingredient. For example, for acetaminophen tablets, USP 24 specifies that in pH 5.8 phosphate buffer, using USP apparatus 2 (paddles) at 50 rpm, at least 80% of the acetaminophen contained in the dosage form is released therefrom within 30 minutes after dosing, and for ibuprofen tablets, USP 24 specifies that in pH 7.2 phosphate buffer, using USP apparatus 2 (paddles) at 50 rpm, at least 80% of the ibuprofen contained in the dosage form is released therefrom within 60 minutes after dosing. See USP 24, 2000 Version, 19 - 20 and 856 (1999).

In another embodiment of this invention at least one active ingredient contained within the dosage form exhibits a delayed and sustained release profile. By "delayed then sustained release profile" it is meant that the release of that particular active ingredient from the dosage form is delayed for a pre-determined time after ingestion by the patient, and the delay period ("lag time") is followed by sustained (prolonged, extended, or retarded) release of that active ingredient. The shell of the present invention provides for the delay period, and is preferraby substantially free of the active to be released in a delayed then sustained manner. In such embodiments, the delayed then sustained release active ingredient is preferrably contained within the core. In such embodiments the core may function for example as an eroding matrix or a diffusional matrix, or an osmotic pump. In embodiments in which the core or a portion thereof functions as a diffusional matrix through which active ingredient is liberated in a sustained, extended, prolonged, or retarded manner, the core portion preferably comprises a release-modifying excipient selected from combinations of insoluble edible materials and pore-formers. Alternately, in such embodiments in which the core portion is prepared by molding, the thermal-reversible carrier may function by dissolving and forming pores or channels through which the active ingredient may be liberated. In embodiments in which the core or a portion thereof functions as an eroding matrix from which dispersed active ingredient is liberated in a sustained, extended, prolonged, or retarded manner, the core portion preferably comprises a release-modifying compressible or moldable excipient selected from swellable erodible hydrophilic materials, pH-dependent polymers, and combinations thereof.

In one embodiment, the shell is not a compression coating applied to the core.

This invention will be illustrated by the following examples.

Example 1

Dosage forms according to the invention, comprising a core within a shell, were prepared as follows.

The following ingredients were used to make the shells:

Table A

| Shell | Trade Name | Manufacturer | Weight percent | Mg/Tablet |
|---|---|---|---|---|
| Polyethylene Glycol 3350 | Carbowax® | Union Carbide Corporation, Danbury, CT | 45.0 | 250 |
| Polyethylene Oxide (MW 200,000) | Polyox® WSR N-80 | Union Carbide Corporation, Danbury, CT | 15.0 | 83 |
| Shellac Powder | Regular bleached shellac | Mantrose-Haeuser Company, Atteboro, MA | 20.0 | 111 |

(continued)

| Shell | Trade Name | Manufacturer | Weight percent | Mg/Tablet |
|---|---|---|---|---|
| Triethyl Citrate | | Morflex, Inc., Greensboro, NC | 10.0 | 56 |
| Croscarmellose Sodium | Ac-Di-Sol® | FMC Corporation, Newark, DE | 10.0 | 56 |

The shell material was prepared as follows: a beaker was submersed in a 70°C water bath (Ret digi-visc; Antal-Direct, Wayne, PA). Polyethylene glycol (PEG) 3350 was added to the beaker and was mixed with a spatula until all PEG was melted. Shellac powder, which was screened through 420 micrometre (40-mesh) screen, was added to the molten PEG and was mixed until all powder was dispersed. Triethyl citrate was added to the molten PEG and was mixed for 1 minute. Polyethylene oxide (PEO) (MW=200,000) was added and was mixed for 10 minutes. Croscarmellose sodium was added and was mixed for 2 minutes. The shell material was provided in flowable form.

The following commercially available product was used as the core:

Table B

| Tablet | Trade Name | Manufacturer | Weight percent | Mg/Tablet |
|---|---|---|---|---|
| Pseudoephedrine HCL core tablet | Nasal decongestant tablet 30 mg | CVS Pharmacy, Inc., Woonsocket, RI | 23.0 | 165 |

The shell was molded according to the following process: a laboratory scale (round, tablet-shaped, 1.11 cm [0.4375"] diameter) stainless steel mold assembly was used to apply the shells to the cores. The mold assembly comprised a lower mold portion and an upper mold portion, with no temperature control. 350 to 450 mg of the molten shell material was introduced into the mold cavity formed by the lower mold portion. A pseudoephedrine HCL core tablet (Table B) was then inserted into the mold cavity. Additional molten shell material was added to fill the mold cavity and the upper mold portion was manually applied to form a molded tablet. After 10 seconds, the upper mold portion was removed, and the molded dosage form was ejected from the lower mold portion.

Reference Example 2

Dosage forms according to the invention, comprising a core within a shell, were prepared as follows.

The following ingredients were used to make the shells:

Table C

| Shell | Trade Name | Manufacturer | Weight percent | Mg/Tablet) |
|---|---|---|---|---|
| Lauroyl Macrogol-32 Glycerides | Gelucire 50/13 | Gattefosse Corp., Westwood, NJ | 70.0 | 751 |
| Lauroyl Macrogol-32 Glycerides | Gelucire 44/14 | Gattefosse Corp., Westwood, NJ | 30.0 | 322 |

The shell material was prepared in the following manner: a beaker was submersed in a water bath (Ret digi-visc; Antal-Direct, Wayne, PA) where the water temperature was set at 70°C. Lauroyl Macrogol-32 glycerides were added to the beaker and were mixed with a spatula until all the glycerides were melted. The shell material was provided in flowable form.

The shell material was applied to the cores of Example 1, using the laboratory scale mold assembly of Example 1, in the following manner: 700 to 800 mg of the molten shell material (Table C) was introduced into the mold cavity formed by the lower mold portion. A pseudoephedrine HCL core tablet (Example 1) was then inserted into the mold cavity. Additional molten mixture was added to fill the mold cavity and the upper mold portion was manually applied to form a molded tablet. After 10 seconds, the upper mold portion was removed, and the molded dosage form was ejected from the lower mold portion.

Example 3

Dosage forms according to the invention, comprising a core within a shell, were prepared as follows.

The following ingredients were used to make the shells:

Table D

| Shell | Trade Name | Manufacturer | Weight percent | Mg/Tablet |
|---|---|---|---|---|
| Polyethylene Glycol 3350 | Carbowax® | Union Carbide Corporation, Danbury, CT | 65.0 | 430 |
| Hydroxypropyl Methylcellulose | Methocel K4M Perm CR | Dow Chemical Co., Midland, MI | 25.0 | 165 |
| Hydroxpropylcellulose | Klucel EXAF Pharm | Hercules Incorporated, Wilmington, DE | 10.0 | 66 |

The shell material was prepared in the following manner: a beaker was submersed in a water bath (Ret digi-visc; Antal-Direct, Wayne, PA) where the water temperature was set at 70°C. PEG 3350 was added to the beaker and was mixed with a spatula until all PEG was melted. Hydroxypropyl methylcellulose and hydroxpropylcellulose were added to the molten PEG and mixed for 10 minutes. The shell material was provided in flowable form.

The following ingredients were used to make the cores:

Table E

| Granulation | Trade Name | Manufacturer | Weight percent | Mg/Tablet |
|---|---|---|---|---|
| Ibuprofen | | Albemarle Corp., Orangeburg, SC | 93.24 | 200 |
| Sodium Starch Glycolate | Explotab | Mendell, A Penwest Co., Patterson, NY | 5.59 | 12 |
| Colloidal Silicon Dioxide | Cab-O-Sil | Cabot Corp., Tuscola, IL | 0.23 | 0.5 |
| Magnesium Stearate | | Mallinckrodt Inc., St. Louis, MO | 0.93 | 2 |

The cores were made in the following manner: Ibuprofen was screened through a 840 micrometre (#20 mesh) screen and was added to a plastic bag. Sodium starch glycolate was screened through a 590 micrometre (#30 mesh) screen and was added to the plastic bag. The powder was blended by manual shaking for 2 minutes. Half of the powder was removed from the first plastic bag and was added to a second plastic bag containing colloidal silicon dioxide and magnesium stearate. The powder in the second bag was then blended by manual shaking for 1 minute and was passed a 840 micrometre (#20 mesh) screen. The resultant powder blend was added to the first bag and further blended by manual shaking for 1 minute. The blend was then compressed into tablets using a Manesty Beta-press (Thomas Engineering, Inc., Hoffman Estates, IL) fitted with round, concave punch and die unit having 0.79 cm (0.3125") diameter.

The shell material was applied to the cores, using a laboratory scale metal mold assembly (1.29 cm [0.5065"], round), made from a lower mold assembly portion comprising a lower mold cavity, and an upper mold assembly portion comprising an upper mold cavity. The dosage form was prepared in the following manner 400 to 450 mg of the molten shell material (Table D) was introduced into the lower mold cavity formed by the lower mold assembly. An ibuprofen core tablet as described above was then inserted into the lower mold cavity. Additional molten shell material was then introduced into the upper mold cavity formed by the upper mold assembly. The upper mold assembly was mated with the lower mold assembly to form a dosage form. After 60 seconds, the upper and lower mold assemblies were separated, and the dosage form was removed from the mold.

Example 4

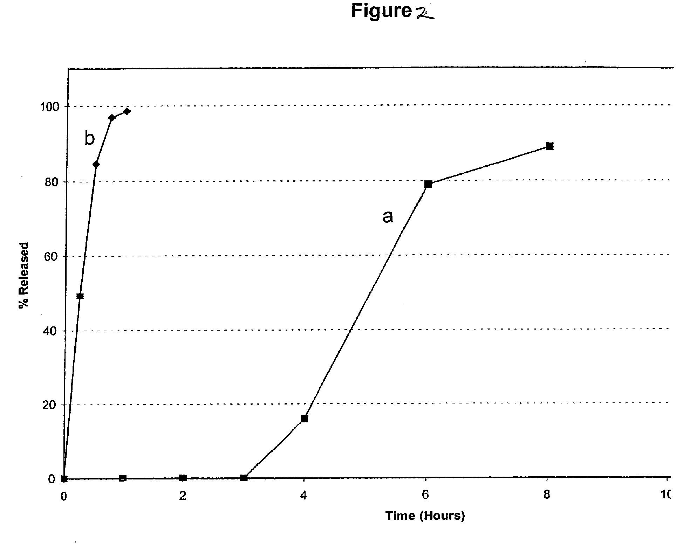

The release profiles for the active ingredients contained in the dosage forms of Examples 1 - 3 were compared with those of the same active ingredients from other dosage forms. Results are shown in Figures 2 - 4.

All dissolution testing was performed according to the following method:

Apparatus: USP Type I apparatus (Basket, 100 RPM).

Media: 0.1 N HCL and pH 5.6 phosphate buffer at 37°C. The pH of the buffer was switched from 0.1N HCL to pH 5.6 phosphate buffer at the 2 hour time point.

Time points: Samples were tested at 0.5, 1, 2, 3, 4, 6, and 8 hours for pseudoephedrine HCL.

Analysis: Dissolution samples were analyzed for pseudoephedrine HCL versus a standard prepared at the theoretical concentration for 100 percent released of each compound. Samples were analyzed using a HPLC equipped with a Waters® 717 Autoinjector and a Waters® 486 UV detector set at a wavelength of 214 nm. The mobile phase was prepared using 55 percent acetonitrile and 45 percent 18 mM Potassium phosphate buffer. The injection volume was 50 μL with a run time of approximately 8 minutes and a pump flow of 2.0 mL/min. The column used was a Zorbax® 300-SCX (4.6mm x 25 cm).

Fig. 2 depicts the percent release of active ingredient (pseudoephedrine HCL) vs. time (hours) for the dosage form of Example 1, as well as the percent release of pseudoephedrine vs. time (hours) for a commercially available immediate release dosage form (Nasal decongestant tablet made by CVS Pharmacy, Inc. - Table B). Curve (a) shows the release of pseudoephedrine HCl from the dosage form of Example 1. Curve (b) shows the release of pseudoephedrine HCl from the immediate release comparitor tablet. As shown in Fig. 2, the dosage form of Example 1 exhibited a delay of about 3 hours to the onset of release of active ingredient.

Fig. 3 depicts the percent release of active ingredient (pseudoephedrine HCL) vs. time (hours) from the dosage form of Example 2, as well as the percent release of pseudoephedrine vs. time (hours) from a commercially available immediate release tablet (Sudafed®). As shown in Fig. 3, the dosage form of Example 2 exhibited a delay of about 4 hours to the onset of release of active ingredient. Curve (a) shows the release rate of pseudoephedrine HCL of this invention. Curve (d) was derived from the commercially immediate release dosage forms of Sudafed® tablet (containing pseudoephedrine HCL).

Fig. 4 depicts the percent release of active ingredient (Ibuprofen) vs. time (hours) for the dosage form of Example 3. As shown in Fig. 4, the dosage form of Example 3 exhibited a delay of about 8 hours to the onset of release of active ingredient.

Example 5

Dosage forms according to the invention, comprising a compressed core within a molded shell, are made in a continuous process using an apparatus comprising a compression module and a thermal cycle molding module linked in series via a transfer device as described at pages 14-16 of copending U.S. Application Serial No. 09/966,939. The shells are made of a shell flowable material comprising the ingredients shown in Table D above and prepared in flowable form as described in Example 3. The cores are made of the ingredients shown in Table E above and prepared as a powder as described in Example 3. The cores are first compressed on a compression module as described in copending U.S. Application Serial No. 09/966,509 at pages 16-27. The compression module is a double row, rotary apparatus, comprising a fill zone, insertion zone, compression zone, ejection zone, and purge zone as shown in Figure 6 of U.S. Application Serial No. 09/966,509. The dies of the compression module are filled using vacuum assistance, with mesh screen filters located in die wall ports of each die.

The cores are transferred from the compression module to the thermal cycle molding module via a transfer device. The transfer device has the structure shown as 300 in Figure 3 and described on pages 51-57 of copending U.S. Application Serial No. 09/966,414. It comprises a plurality of transfer units 304 attached in cantilever fashion to a belt 312 as shown in Figures 68 and 69. The transfer device rotates and operates in sync with the compression and thermal cycle molding modules to which it is coupled. Transfer units 304 comprise retainers 330 for holding the cores as they travel around the transfer device.

The thermal cycle molding module has the general configuration shown in Figure 3 and pages 27-51 of copending U.S. Application Serial No. 09/966,497, which depicts a thermal cycle molding module 200 comprising a rotor 202 around which a plurality of mold units 204 are disposed. The thermal cycle molding module includes reservoir 206 (see Figure 4) for holding the shell flowable material. In addition, the thermal cycle molding module is provided with a temperature control system for rapidly heating and cooling the mold units. Figures 55 and 56 of pending U.S. Application Serial No. 09/966,497 depict the temperature control system 600.

The thermal cycle molding module is of the type shown in Figure 28A of copending U.S. Application Serial No. 09/966,497. The mold units 204 of the thermal cycle molding module comprise upper mold assemblies 214, rotatable center mold assemblies 212 and lower mold assemblies 210 as shown in Figure 28C. Cores are continuously transferred to the mold assemblies, which then close over the cores.

Coating is performed in two steps, first and second portions of the shell portions being applied separately as shown in the flow diagram of Figure 28B of copending U.S. Application Serial No. 09/966,497. In a first step, a first portion of shell flowable material, heated to a flowable state in reservoir 206, is injected into the mold cavities created by the closed mold assemblies. The temperature of the first portion is then decreased, hardening it over half the core. The mold assemblies separate, the center mold assembly rotates, and then the mold assemblies again close. In a second step, the second portion of the shell flowable material, heated to a flowable state in reservoir 206, is injected into the mold cavities. The temperature of the second portion is then decreased, hardening it over the other half of the core. The mold assemblies separate, and the finished dosage forms are ejected from the apparatus.

## Claims

**1.** A dosage form comprising:

> (a) a core comprising at least one active ingredient; and
> (b) a molded shell which surrounds the core,

wherein the shell provides a predetermined time delay of greater than one hour for the onset of dissolution of the active ingredient upon contacting of the dosage form with a liquid medium and the delay is independent of the pH of the liquid medium and wherein the weight of the shell is at least 50 percent of the weight of the core; the shell has a thickness from 500 to 4000 micrometres; the shell has a pore volume of less than 0.02 cm$^3$/g in the pore diameter range 0.5 to 5.0 micrometres; and wherein the shell comprises at least 30% by weight of a thermal reversible carrier, selected from the group consisting of polyethylene glycol, polyethylene oxide and copolymers and combinations thereof.

**2.** The dosage form of Claim 1, which additionally comprises an outer coating which covers at least a portion of the shell, and the outer coating comprises at least one active ingredient which may be the same or different than the active ingredient contained in the core.

**3.** The dosage form of Claim 1, in which the core is a compressed tablet.

**4.** The dosage form of Claim 1, in which the core is prepared by molding.

**5.** The dosage form of Claim 1, in which the shell is not a compression coating applied to the core.

**6.** The dosage form of Claim 1, in which the shell is prepared using a solvent-free molding process.

## Patentansprüche

**1.** Dosierform umfassend:

> a) einen Kern mit mindestens einem Wirkstoff; und
> b) einen Formmantel, der den Kern umgibt und

eine vorbestimmte zeitliche Verzögerung von mehr als einer Stunde für den Beginn des Auflosens des Wirkstoffes bereitstellt, nachdem die Dosierform mit einem flüssigen Medium in Kontakt gekommen ist, wobei die Verzögerung unabhängig vom pH-Wert des flüssigen Mediums ist und der Formmantel mindestens 50 % des Gewichts des Kernes aufweist; wobei der Formmantel eine Dicke von 500 bis 4000 μm aufweist; wobei der Formmantel ein Porenvolumen von weniger als 0,02 cm$^3$/g im Porendurchmesserbereich von 0,5 bis 5,0 μm aufweist; und wobei der Formmantel mindestens 30 Gewichts- % eines reversiblen Wärmeträgers umfaßt, der aus einer Gruppe aus Polyethylenglykol, Polyethylenoxid und Copolymeren davon und Kombinationen davon gewählt ist.

**2.** Dosierform nach Anspruch 1, die zusätzlich eine Außenschicht aufweist, die mindestens einen Abschnitt des Formmantels bedeckt und mindestens einen Wirkstoff umfaßt, der mit dem im Kern enthaltenden Wirkstoff identisch sein oder sich von diesem unterscheiden kann.

**3.** Dosierform nach Anspruch 1, bei welcher der Kern eine komprimierte Tablette ist.

**4.** Dosierform nach Anspruch 1, bei der der Kern durch Formen hergestellt ist.

**5.** Dosierform nach Anspruch 1, bei der der Formmantel keine an dem Kern angebrachte Kompressionsschicht ist.

**6.** Dosierform nach Anspruch 1, bei der der Formmantel durch einen lösungsmittelfreien Formprozeß hergestellt ist.

**Revendications**

**1.** Forme pharmaceutique comprenant :

(a) un noyau comprenant au moins un ingrédient actif ; et
(b) une coque moulée qui entoure le noyau,

dans laquelle la coque produit un retard prédéterminé de plus d'une heure avant le début de la dissolution de l'ingrédient actif lors du contact de la forme pharmaceutique avec un milieu liquide et le retard est indépendant du pH du milieu liquide et dans laquelle le poids de la coque est au moins 50 pour cent du poids du noyau ; la coque a une épaisseur de 500 à 4000 micromètres ; la coque a une porosité de moins de 0,02 cm$^3$/g dans la plage de diamètre de pore de 0,5 à 5,0 micromètres ; et dans laquelle la coque comprend au moins 30 % en poids d'un support thermique réversible, choisi dans le groupe constitué du polyéthylèneglycol, du poly(oxyde d'éthylène) et de copolymères et combinaisons de ceux-ci.

**2.** Forme pharmaceutique selon la revendication 1, qui comprend en outre un revêtement externe qui recouvre au moins une partie de la coque, et le revêtement externe comprend au moins un ingrédient actif qui peut être le même ou différent de l'ingrédient actif contenu dans le noyau.

**3.** Forme pharmaceutique selon la revendication 1, dans laquelle le noyau est un comprimé.

**4.** Forme pharmaceutique selon la revendication 1, dans laquelle le noyau est préparé par moulage.

**5.** Forme pharmaceutique selon la revendication 1, dans laquelle la coque n'est pas un revêtement de compression appliqué au noyau.

**6.** Forme pharmaceutique selon la revendication 1, dans laquelle la coque est préparée en utilisant un procédé de moulage sans solvant.

Fig. 1

# Figure 2

# Figure 3

Figure 4

REFERENCES CITED IN THE DESCRIPTION

Patent documents cited in the description

- US 4576604 A
- US 5738874 A
- US 6294200 B
- WO 9951209 A
- US 5464633 A
- US 5415868 A
- US 5824338 A
- US 5100675 A
- US 5558879 A
- US 5807579 A
- EP 0279682 A
- US 4906478 A
- US 5275822 A
- US 6103260 A
- US 4173626 A
- US 4863742 A

- US 4980170 A
- US 4984240 A
- US 5286497 A
- US 5912013 A
- US 6270805 A
- US 6322819 A
- US 966509 A
- US 5427614 A
- US 5146730 A
- US 5459983 A, Banner Gelatin Products Corp.
- US 966450 A
- US 966497 A
- US 966939 A
- US 966414 A

Non-patent literature cited in the description

- **G. MAFFIONE et al.** High-Viscosity HPMC as a Film-Coating Agent. *Drug Development and Industrial Pharmacy,* 1993, vol. 19 (16), 2043-2053
- Compression-coated and layer tablets. **GUNSEL et al.** Pharmaceutical Dosage Forms - Tablets. rev. and expanded Marcel Dekker) Inc, 247-284

- **K. FEGLEY ; C. VESEY.** *The Effect of Tablet Shape on the Perception of High Gloss Film Coating Systems,* 18 March 2002, www.colorcon.com